# EUROPÄISCHE PATENTANMELDUNG

(11) **EP 1 358 853 A2**
(43) Veröffentlichungstag der Anmeldung: **05.11.2003**
(21) Anmeldenummer: 03006966.0
(22) Anmeldetag: 27.03.2003
(51) Int. Cl.: A61B 18/14

(54) **Ablationsvorrichtung für Herzgewebe, insbesondere zur Erzeugung linearer Läsionen zwischen zwei Gefässmündungen im Herzen**

(30) Priorität: 24.04.2002 DE 10218426
(71) Anmelder: BIOTRONIK Mess- und Therapiegeräte GmbH & Co Ingenieurbüro Berlin, 12359 Berlin (DE)
(72) Erfinder: Geistert, Wolfgang, Dr., 79618 Rheinfelden (DE)
(74) Vertreter: Hübner, Gerd, Dipl.-Phys.

(57) **Zusammenfassung**

Eine Ablationsvorrichtung für Herzgewebe, insbesondere zur Erzeugung linearer Läsionen zwischen zwei Gefäßmündungen (6,15) im Herzen, umfasst
- einen Ablationskatheter (2), der vor seinem distalen Ende mit einem sich über eine bestimmte Katheterlänge erstreckenden Ablationsapplikator (8) versehen ist,
- einen steuerbaren Positionierkatheter (10), an dem der Ablationskatheter (2) relativ dazu in Längsaxialrichtung verschieblich geführt ist, und
- einen distalen Endabschnitt (14) am Positionierkatheter (10), proximal von dem die Führung (11) des Ablationskatheters (2) durch den Positionierkatheter (10) endet und der Ablationskatheter (2) mit seinem Ablationsapplikator (8) frei beweglich ist, wobei durch längsaxiales Verschieben der beiden Katheter (2, 10) zueinander die außerhalb der Führung (11) liegende wirksame Ablationslänge (AL) des Ablationsapplikators (8) variierbar ist.

## Beschreibung

Die Erfindung betrifft eine Ablationsvorrichtung für Herzgewebe, insbesondere zur Erzeugung linearer Läsionen zwischen zwei Gefäßmündungen im Herzen nach dem Oberbegriff des Patentanspruches 1.

Zum Hintergrund der Erfindung ist festzuhalten, dass die Katheterablation eine vermehrt eingesetzte Therapie zur Behandlung bestimmter Arrhythmien ist. Dabei wird an einer bestimmten Stelle im Herzmuskelgewebe mit Hilfe des Ablationsapplikators des Katheters eine Läsion - also eine Gewebedenaturierung in der Art einer Gewebevernarbung - erzeugt, um dort die für die Arrhytmien verantwortlichen, fehlerhaften elektrischen Reizleitungsbahnen zu unterbrechen. Die Energie-Einbringung in das Herzmuskelgewebe über den Ablationsapplikator erfolgt dabei in aller Regel über Ablationselektroden, die mit Hochfrequenzstrom arbeiten. Zur Ablation können ferner andere Energieformen, wie Mikrowellenenergien, Hochspannungs-Gleichstrom oder prinzipiell andere Denaturierungsmechanismen, wie Kälte oder Chemikalien (z.B. Alkohol) eingesetzt werden. Der Begriff "Ablationsapplikator", wie er in der vorliegenden Anmeldung, insbesondere auch in Verbindung mit dem Erfindungsgegenstand, benutzt wird, soll grundsätzlich alle genannten Ablationsmöglichkeiten umfassen, wobei Ablationselektroden die gängigste Variante darstellen.

Für eine besondere Art der Behandlung des sogenannten Vorhof-Flimmerns ist es notwendig, die Mündungsöffnungen der Pulmonalvenen in den linken Vorhof durch lineare Läsionen zu verbinden. Dies gestaltet sich mit üblichen Ablationskathetern schwierig, da die gewünschten Ablationspositionen nur schwer erreichbar sind und kaum stabil gehalten werden können.

Zur Lösung dieser Problematik ist aus der WO 98 49 957 A1 bzw. der US 6 164 283 A eine spezielle Ablationsanordnung bekannt, wie sie dem Oberbegriff des Anspruches 1 entspricht. Diese Ablationsvorrichtung weist dementsprechend einen steuerbaren Ablationskatheter auf, der vor seinem distalen Ende mit einem sich über eine bestimmte Katheterlänge erstreckenden Ablationsapplikator versehen ist. In diesem aktiven Teil wird der Ablationskatheter mittels zweier Führungsdrähte im Bereich der Pulmonalvenen-Mündungsöffnungen positioniert und stabil gehalten. Dabei wird der erste Führungsdraht axial durch den distalen Abschnitt des Katheters geschoben, während der zweite Führungsdraht proximal vom Ablationsaplikator schräg durch den Katheterkörper hindurchläuft.

Ein wesentlicher Nachteil der vorbekannten Ablationsvorrichtung liegt darin, dass die aktive Länge des Ablationsapplikators durch den Abstand zwischen der Eintrittsöffnung für den ersten Führungsdraht am distalen Ende und der Position, an der der zweite Führungsdraht in proximalerer Position den Katheterkörper durchdringt, fest vorgegeben ist. In der anatomischen Praxis ist jedoch der Abstand zwischen den Pulmonalvenen-Mündungsöffnungen von Patient zu Patient verschieden, so dass zur Behandlung verschiedener Patienten entweder ein Satz von Ablationskathetern mit unterschiedlich langen Ablationsapplikatoren bereitgestellt oder eine nicht optimale Anpassung der Läsion an die anatomischen Gegebenheiten in Kauf genommen werden müssen.

Der Erfindung liegt dementsprechend die Aufgabe zugrunde, eine Ablationsvorrichtung der vorstehend erörterten Art so zu verbessern, dass sie in flexibler Weise an unterschiedliche anatomische Bedingungen optimal anpassbar ist.

Diese Aufgabe wird durch die im Kennzeichnungsteil des Anspruches 1 angegebenen Merkmale gelöst. Demnach ist neben dem eigentlichen Ablationskatheter mit seinem Ablationsapplikator ein steuerbarer Positionierkatheter vorgesehen, an dem der Ablationskatheter relativ dazu in Längsaxialrichtung verschiebbar geführt ist. Diese Führung endet proximal vor einem vorzugsweise steuerbaren distalen Endabschnitt am Positionierkatheter, so dass der Ablationskatheter mit seinem Ablationsapplikator in diesem über die Führung hinausstehenden Bereich frei beweglich ist. Ferner wird durch diese Konstruktion ermöglicht, das distale Ende des Positionierkatheters in die zweite zugeordnete Gefäßmündung über eine bestimmte Distanz einzuführen. Durch ein längsaxiales Verschieben der beiden Katheter zueinander ist die außerhalb der Führung liegende, wirksame Ablationslänge des Ablationsapplikators dann variierbar.

Der Positionierkatheter kann selbst mit Hilfe eines Zugdrahtes steuerbar oder mit Hilfe eines separaten Führungsdrahtes beziehungsweise -katheters an seine gewünschte Position verbracht werden.

Erfindungsgemäß kann also die Ablationsvorrichtung durch diese Variierung der wirksamen Ablationslänge an die jeweiligen anatomischen Gegebenheiten optimal angepasst werden.

Bevorzugte Ausführungsformen der Ablationsvorrichtung sind in den Unteransprüchen angegeben. Nähere Informationen hierzu sowie weitere Merkmale, Einzelheiten und Vorteile der Erfindung sind der nachfolgenden Beschreibung entnehmbar, in der Ausführungsbeispiele des Erfindungsgegenstandes anhand der beigefügten Zeichnungen näher erläutert werden. Es zeigen:
- Fig. 1: eine schematische Darstellung einer Ablationsvorrichtung in einer ersten Ausführungsform während der Applikation einer Läsion,
- Fig. 2: eine Darstellung analog Fig. 1 einer zweiten Ausführungsform der Ablationsvorrichtung,
- Fig. 3A bis C: ausschnittsweise Seitenansichten der beiden aneinander geführten Katheter der Ablationsvorrichtung gemäß Fig. 1 bzw. 2, und
- Fig. 4A bis C: Schnitte der beiden Katheter gemäß den Schnittlinien IV-IV nach Fig. 3A bis C.

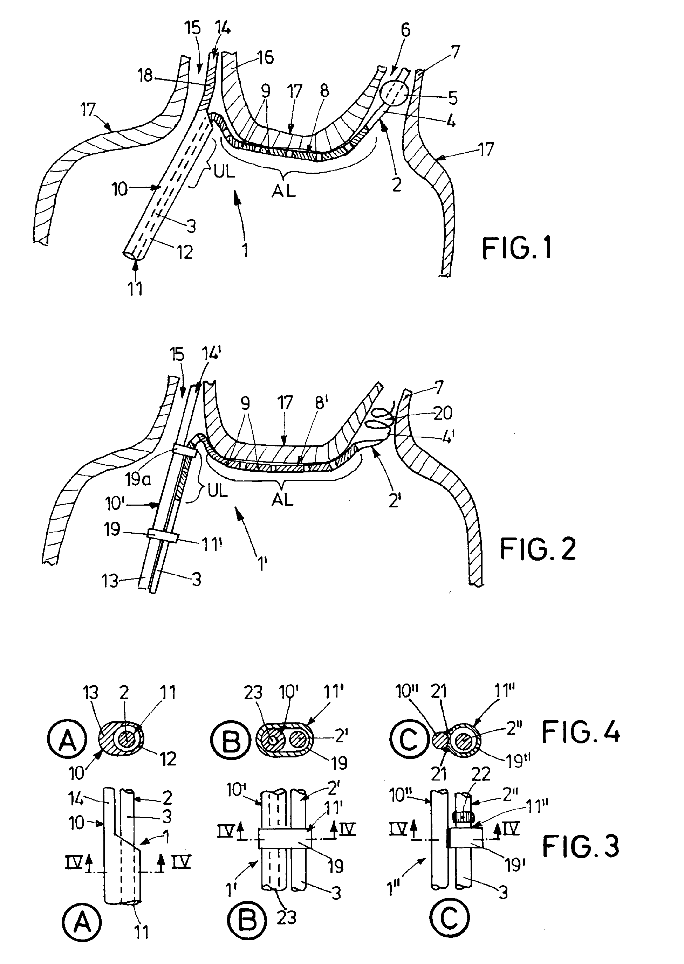

Anhand von Fig. 1 soll der grundsätzliche Aufbau der Ablationsvorrichtung 1 und deren Anwendung im Herzen erläutert werden. So ist ein erster Katheter - der Ablationskatheter 2 - vorgesehen, der in üblicher Weise einen langen dünnen Schaft 3 mit einem proximalen Ende (nicht dargestellt) und einem distalen Ende 4 aufweist.

An seinem distalen Ende 4 weist der Ablationskatheter 2 einen dilatierbaren Ballon 5 als Widerlagereinrichtung auf, der zur Festlegung des Katheters 2 in einer Körperöffnung, wie beispielsweise einer Mündungsöffnung 6 einer Pulmonalvene 7 in das Atrium des Herzens dient.

Proximal vor dem Ballon 5 ist der Ablationskatheter 2 über eine Länge in der Größenordnung von 10 cm mit dem eigentlichen Ablationsapplikator 8 versehen, der beispielsweise aus einer Aneinanderreihung von hochflexiblen Ringelektroden 9 besteht. Über diese Ringelektroden 9 kann Hochfrequenzstrom in noch näher zu erläuternder Weise zur Erzeugung einer Läsion abgegeben werden.

Wie ferner aus Fig. 1 hervorgeht, ist ein zweiter Katheter - der so bezeichnete Positionierkatheter 10- vorgesehen, der analog dem Ablationskatheter mit einem Lumen für einen Führungsdraht oder einen Steuerdraht versehen sein kann und entsprechend über einen Steuerungs- und Deflektionsmechanismus steuerbar ist. Der Positionierkatheter 10 dient generell zur Führung und Positionierung des Ablationskatheters 2. Dazu weist der Positionierkatheter 10 eine mit 11 bezeichnete Führung für den Ablationskatheter 2 auf, die im Ausführungsbeispiel gemäß Fig. 1 durch ein Lumen 12 im Positionierkatheter 10 gebildet ist. Wie aus Fig. 3A und 4A deutlich wird, weist der Positionierkatheter auf dem Großteil seiner Länge einen langovalen Querschnitt auf, in dem das Lumen 12 an exzentrischer Position integriert ist. Dieser im Querschnitt langovale Schaft 13 setzt sich am distalen Ende der Führung 11 in einen distalen Endabschnitt 14 fort, der mit den genannten Mitteln steuerbar ist. Durch längsaxiales Verschieben der beiden Katheter 2, 10 zueinander ist die außerhalb der Führung 11 liegende, wirksame Ablationslänge AL variierbar.

Anhand von Fig. 1 ist das Anbringen einer linearen Läsion zwischen der erwähnten Mündungsöffnung 6 der Pulmonalvene 7 und der benachbarten Mündungsöffnung 15 einer zweiten Pulmonalvene 16 zu erläutern. So wird zuerst der Ablationskatheter 2 mit seinem distalen Ende 4 in die Mündungsöffnung 6 der Pulmonalvene 7 eingeführt, indem er auf dem Positionierkatheter dorthin vorgeschoben wird. Dann wird der Ballon 5 zur Dilatation und zur Fixierung des distalen Endes 4 inflatiert.

Anschließend wird der Positionierkatheter 10 zurückgezogen und zur Mündungsöffnung 15 der zweiten Pulmonalvene 16 manövriert. Dabei wird die wirksame Ablationslänge AL des Ablationskatheters 12 freigelegt. Der distale Endabschnitt 14 des Positionierkatheters 10 wird schließlich soweit in die Pulmonalvene 16 hineingeschoben, dass die wirksame Ablationslänge AL stabil am Gewebe anliegt. Innerhalb der Führung 11 verbleibt bei entsprechender Einstellung die unwirksame Länge UL des Applikators 8. Durch die genaue Anpassung der Ablationslänge AL an die jeweiligen anatomischen Gegebenheiten wird eine saubere und in ihrer Länge exakte Läsion durch Applizieren von Hochfrequenzstrom über den Ablationsapplikator 8 erzeugt. Um einen guten Energieübertrag vom Applikator 8 zur Herzwand 17 zu erzielen, wird dabei der Positionierkatheter 10 in Richtung in die Pulmonalvene 16 hinein geschoben, so dass der Ablationsapplikator 8 mechanisch gegen die Herzwand 17 gedrückt wird.

Wie in Fig. 1 durch eine schematische Schraffur angedeutet ist, kann der Positionierkatheter 10 im Bereich seines distalen Endabschnittes 14 mit einem zusätzlichen Ablationsapplikator 18 versehen sein.

Bei der in Fig. 2 gezeigten Ausführungsform der Ablationsvorrichtung 1' ist einerseits die Führung 11' des Ablationsapplikators 8' am Positionierkatheter 10' anders als in Fig. 1 ausgeführt. So besteht die Führung 11' aus Führungsringen 19, die mit Abstand zueinander am Positionierkatheter 10' aneinandergereiht sind. Der letzte distale Führungsring 19a markiert dabei das Ende der Führung 11', an die sich der führungsfreie distale Endabschnitt 14' des Positionierkatheters 10' anschließt. Wie aus Fig. 3B und 4B deutlich wird, bestehen hier die Führungsringe 19 aus langovalen Schlaufen, die mit dem Positionierkatheter 10' exzentrisch fest verbunden sind.

Im freibleibenden Schlaufenquerschnitt ist der Ablationskatheter 2' geführt. Ferner weist die Ausführungsform gemäß Fig. 3B und 4B im Positionierkatheter 10' ein Lumen 23 für den eingangs erwähnten Führungsdraht auf.

Im Unterschied zur Ausführungsform gemäß Fig. 1 ist beim Ablationskatheter 2' als Fixierung des distalen Endes 4' kein Ballon, sondern eine aufweitbare Wendelspitze 20 vorgesehen. Die Ausformung dieser Wendelspitze 20 kann durch eine übliche Zugvorrichtung oder beispielsweise mittels Ausbildung durch ein Memorymetall erfolgen.

Im übrigen ist die Anwendung und insbesondere Einstellung der wirksamen Ablationslänge AL bei der Ablationsvorrichtung 1' gemäß Fig. 2 zu der Ablationsvorrichtung 1 gemäß Fig. 1 unverändert.

Bei den in Fig. 3C und 4C gezeigten Ausführungsformen der Ablationsvorrichtung 1" sind die Führungsringe 19' nicht als langovale Schlaufen, wie bei der Ausführungsform gemäß Fig. 3B, 4B, sondern als im Querschnitt kreisrunde Ringe ausgebildet, die durch geeignete Verbindung 21 in Form von beispielsweise Kleben oder Schweißen am Positionierkatheter 10' befestigt sind. Der Ablationskatheter 2" kann analog Fig. 1 oder 2 ausgestaltet sein. Wie in Fig. 3C gezeigt ist, markiert auch bei dieser Ausführungsform analog der gemäß Fig. 2, 3B und 4B der letzte Führungsring 19' das Ende der Führung 11" und den Beginn des distalen Endabschnittes 14 des Positionierkatheters 10". Wie ferner in Fig. 3C angedeutet ist, kann der Ablationskatheter 2" vor seinem distalen Ende mit einer Verdickung 22 versehen sein, die ein Herausrutschen dieses Katheters aus der Führung 11" verhindert.

Die Länge der distalen Endabschnitte 14, 14' der Positionierkatheter 10, 10', 10" kann in der Größenordnung zwischen 5 und 50, vorzugsweise zwischen 10 und 30 mm, liegen.

Der Vollständigkeit halber sind noch folgende technische Maßnahmen bei den Ablationsvorrichtungen 1, 1', 1" zu erwähnen, die in den Zeichnungen nicht explizit dargestellt sind:
- Neben den gezeigten Fixierungen des Ablationskatheters 2, 2' mit Hilfe eines Ballons 5 oder einer Wendelspitze 20 können auch andere, beispielsweise hakenförmige oder spiralförmige Fixierungen am distalen Ende der Katheter 2 vorgesehen sein.
- Die Ringelektroden 9 der Ablationsapplikatoren 8, 8' können beispielsweise aus einer flexiblen Metallwendel oder aus einem flexiblen, leitfähigen Kunststoff gefertigt sein, um möglichst hohe Flexibilität zu gewährleisten.
- Das Applikationsmedium des Ablationsapplikators kann im Inneren des Katheters herangeführt und über ein leitfähiges Medium (z.B. Kochsalzlösung) an das Gewebe weitergeleitet werden. Das flüssige Medium kann dabei durch ein zusätzliches Spüllumen zugeführt werden. Die Verwendung eines flüssigen, leitfähigen Mediums hat den Vorteil, dass gleichzeitig das Blut im Bereich der Energieabgabe von der Ablationsstelle weggespült und damit die Koagulationsgefahr verringert werden. Eine Kombination von äußeren Elektroden und einer Spülung ist denkbar.
- Da die Ablation in aller Regel mit einer thermischen Wirkung auf das Herzgewebe einhergeht, kann der durch die Energie erzeugte Effekt durch Thermosensoren in den Kathetern 2 bzw. 10 überwacht werden.

## Patentansprüche

1. Ablationsvorrichtung für Herzgewebe, insbesondere zur Erzeugung linearer Läsionen zwischen zwei Gefäßmündungen (6,15) im Herzen, umfassend
- einen Ablationskatheter (2, 2', 2"), der vor seinem distalen Ende mit einem sich über eine bestimmte Katheterlänge erstreckenden Ablationsapplikator (8, 8') versehen ist,
**gekennzeichnet durch**
- einen steuerbaren Positionierkatheter (10, 10', 10"), an dem der Ablationskatheter (2, 2', 2") relativ dazu in Längsaxialrichtung verschieblich geführt ist, und
- einen distalen Endabschnitt (14, 14') am Positionierkatheter (10, 10', 10"), proximal von dem die Führung (11, 11', 11") des Ablationskatheters (2, 2', 2") **durch** den Positionierkatheter (10, 10', 10") endet und der Ablationskatheter (2, 2', 2") mit seinem Ablationsapplikator (8, 8') frei beweglich ist, , wobei **durch** längsaxiales Verschieben der beiden Katheter (2, 2', 2"; 10, 10', 10") zueinander die außerhalb der Führung (11, 11', 11") liegende, wirksame Ablationslänge (AL) des Ablationsapplikators (8, 8') variierbar ist.

2. Ablationsvorrichtung nach Anspruch 1, **dadurch gekennzeichnet, dass** der Ablationskatheter (2) in einem Lumen (12) des Positionierkatheters (10) verschiebbar geführt ist.

3. Ablationsvorrichtung nach Anspruch 1, **dadurch gekennzeichnet, dass** der Ablationskatheter (2, 2") in einer Reihe von beabstandet am Positionierkatheter (10', 10") angeordneten Führungsringen (19, 19') verschiebbar geführt ist, wobei vor dem distal letzten Führungsring (19a) der führungsfreie Endabschnitt (14') liegt.

4. Ablationsvorrichtung nach einem der vorgenannten Ansprüche, **dadurch gekennzeichnet, dass** der Ablations- (2, 2', 2") und/oder Positionierkatheter (10, 10', 10") mit einem Lumen (23) für einen Führungs- oder Steuerdraht versehen sind.

5. Ablationsvorrichtung nach einem der vorgenannten Ansprüche, **dadurch gekennzeichnet, dass** der Ablationskatheter (2, 2') an seinem distalen Ende (4, 4') mit einer Fixiervorrichtung (5, 20) zum Festlegen in einer kardialen Gefäßmündung (6) versehen ist.

6. Ablationsvorrichtung nach Anspruch 5, **dadurch gekennzeichnet, dass** die Fixiervorrichtung ein deflatierbarer Katheterballon (5) ist.

7. Ablationsvorrichtung nach Anspruch 5, **dadurch gekennzeichnet, dass** die Fixiervorrichtung ein eine distale Wendelspitze (20) ist.

8. Ablationsvorrichtung nach einem der vorgenannten Ansprüche, **dadurch gekennzeichnet, dass** der distale Endabschnitt (14, 14') des Positionierkatheters (10, 10') eine Länge von 5 bis 50 mm, vorzugsweise 10 bis 30 mm, aufweist.

9. Ablationsvorichtung nach einem der vorgenannten Ansprüche, **dadurch gekennzeichnet, dass** der distale Endabschnitt (14, 14') des Positionierkatheters (10, 10') mit einem zusätzlichen Ablationsapplikator (18) versehen ist.

10. Ablationsvorrichtung nach einem der vorhergehenden Ansprüche, **dadurch gekennzeichnet, dass** der distale Endabschnitt (14, 14') selbständig steuerbar ist.
